(19) Europäisches Patentamt
European Patent Office
Office européen des brevets

(11) Veröffentlichungsnummer: **0 112 584**
**A2**

(12) # EUROPÄISCHE PATENTANMELDUNG

(21) Anmeldenummer: **83113153.7**

(22) Anmeldetag: **27.12.83**

(51) Int. Cl.³: **C 07 C 97/07**, C 07 D 295/08, C 07 D 295/10, A 61 K 31/645 // C07C57/58, C07C57/60, C07F9/54

(30) Priorität: **24.12.82 DE 3248094**

(43) Veröffentlichungstag der Anmeldung: **04.07.84**
**Patentblatt 84/27**

(84) Benannte Vertragsstaaten: **AT BE CH DE FR GB IT LI LU NL SE**

(71) Anmelder: **GÖDECKE AKTIENGESELLSCHAFT,**
**Salzufer 16, D-1000 Berlin 10 (DE)**

(72) Erfinder: **Satzinger, Gerhard, Dr., Im Mattenbühl 7,**
**D-7809 Denzlingen (DE)**
Erfinder: **Fritschi, Edgar, Dr., Am Scheuerwald 2,**
**D-7811 St. Peter (DE)**
Erfinder: **Herrmann, Manfred, Dr., Wolfweg 25,**
**D-7811 St. Peter (DE)**

(54) **7H-Dibenzo(a,c)cyclohepten-5-on(7)-Derivate, Verfahren zu deren Herstellung und Arzneimittel zur Behandlung psychischer Erkrankungen und von Magen-Darmgeschwüren.**

(57) Die Erfindung betrifft 7H-Dibenzo(a,c)cyclohepten-5-on(7)-Derivate der allgemeinen Formel I

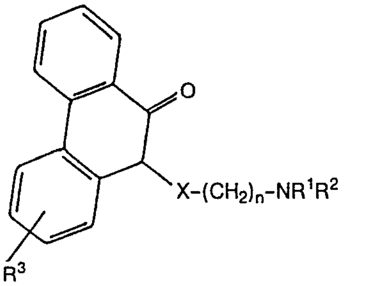

(I),

worin $R^1$ und $R^2$ gleich oder verschieden sein können und ein Wasserstoffatom, eine Alkylgruppe mit 1–3 Kohlenstoffatomen oder zusammen mit dem Stickstoffatom an das sie gebunden sind, einen heterocyclischen Ring mit 3–6 Kohlenstoffatomen,

n die Zahl 2 oder 3

$R^3$ ein Wasserstoffatom oder ein Halogenatom und

X entweder eine Methylengruppe oder ein Sauerstoffatom bedeutet

sowie deren pharmakologisch verträgliche Salze mit anorganischen oder organischen Säuren.

Die Erfindung betrifft weiterhin Analogieverfahren zu deren Herstellung sowie Arzneimittel für die Behandlung von psychischen Erkrankungen sowie von Magen- und Darmgeschwüren, welche mindestens eine Verbindung der allgemeinen Formel I als Wirkstoff enthalten.



COMPLETE DOCUMENT

Beschreibung

Die Erfindung betrifft neue Verbindungen der allgemeinen Formel I

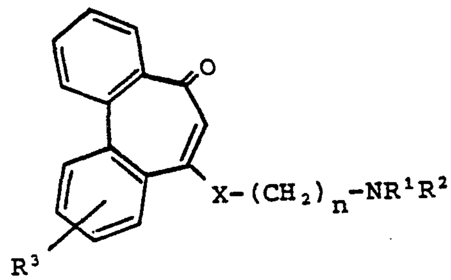

(I),

worin $R^1$ und $R^2$ gleich oder verschieden sein können und ein Wasserstoffatom, eine Alkylgruppe mit 1 - 3 Kohlenstoffatomen oder zusammen mit dem Stickstoffatom an das sie gebunden sind, einen heterocyclischen Ring mit 3 - 6 Kohlenstoffatomen,

n die Zahl 2 oder 3

$R^3$ ein Wasserstoffatom oder ein Halogenatom und

X entweder eine Methylengruppe oder ein Sauerstoffatom bedeutet

sowie deren pharmakologisch verträgliche Salze mit anorganischen oder organischen Säuren.

Die Erfindung betrifft weiterhin ein Verfahren zur Herstellung von Verbindungen der allgemeinen Formel I, sowie von deren pharmakologisch verträglichen Salzen, das dadurch gekennzeichnet ist, daß man eine Verbindung der allgemeinen Formel II

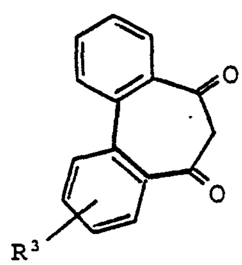

(II),

in welcher $R^3$ die oben angegebene Bedeutung hat

in an sich bekannter Weise entweder

a) mit Grignard-Verbindungen der allgemeinen Formel III

$$\text{Hal Mg-}(CH_2)_n\text{-}NR^1R^2 \quad \text{(III)},$$

in welcher Hal ein Halogenatom bedeutet und n, $R^1$ und $R^2$ die obengenannte Bedeutung haben oder

b) mit Verbindungen der allgemeinen Formel IV

$$(C_6H_5)_3P{=}CH\text{-}(CH_2)_{n-1}\text{-}NR^1R^2 \quad \text{(IV)}$$

in welcher n, $R^1$ und $R^2$ die obengenannte Bedeutung haben oder

c) für den Fall, daß X eine Methylengruppe sein soll, indem man Verbindungen der allgemeinen Formel V

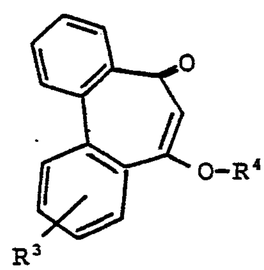

(V),

in welcher $R^3$ die obengenannte Bedeutung hat und $R^4$ einen Alkylrest mit 1 - 3 Kohlenstoffatomen bedeutet mit Verbindungen der allgemeinen Formel IV umsetzt oder

d) für den Fall, daß X ein Sauerstoffatom sein soll, indem man Verbindungen der allgemeinen Formel II zunächst mittels starker Basen in die entsprechenden Enolate überführt und diese mit Verbindungen der allgemeinen Formel VI

$$\text{Hal'-}(CH_2)_n\text{-}NR^1R^2 \quad \text{(VI)}$$

in welcher Hal' ein Halogenatom bedeutet und $R^1$ und $R^2$ die oben angegebene Bedeutung hat,

umsetzt und gewünschtenfalls anschließend mittels anorganischen oder organischen Säuren in deren pharmakologisch verträgliche Salze überführt.

Ein weiterer Gegenstand der Erfindung sind Arzneimittel, welche als Wirkstoff mindestens eine Verbindung der allgemeinen Formel I oder deren pharmakologisch verträgliche Salze sowie gewünschtenfalls Hilfs- und Zusatzstoffe enthalten.

Die Verfahren a) bis d) werden zweckmäßigerweise bei Temperaturen zwischen 20 °C und 60 °C, vorzugsweise um 30 °C durchgeführt. Als Reaktionsmedium verwendet man vorteilhafterweise ein inertes organisches Lösungsmittel wie z.B. Diethylether, Benzol, Toluol, Xylol, Tetrahydrofuran oder Dimethylformamid. Die so erhaltenen neuen Verbindungen der Formel I können in an sich bekannter Weise isoliert und gereinigt werden.

Die in den Verfahren als Ausgangsprodukte verwendeten Verbindungen der Formel II - V sind zum Teil bekannt. Soweit sie nicht in der Literatur erwähnt sind, wird ihre Herstellung beschrieben.

Heterocyclische Ringe im Rahmen der Erfindung sind gesättigte Fünf- Sechs, oder Siebenringe, welche 3 - 6 Kohlenstoffatome und im übrigen als Heteroatome Stickstoff, Schwefel oder Sauerstoff enthalten können. Im Falle von Stickstoffheterocyclen mit freien Valenzen am Stickstoff sind diese durch verzweigte oder geradkettige niedere Alkylgruppen bevorzugt solche, mit 1 - 3 Kohlenstoffatomen substituiert.

Bevorzugte Ringe sind der Piperidin-, der Morpholin- und der N-Alkylpiperazin-Ring, insbesondere der N-Methylpiperazin-Ring.

Von den nicht ringgeschlossenen Resten $R^1$ und $R^2$ sind Wasserstoffatome sowie Methyl- und Ethylreste bevorzugt. n bedeutet bevorzugt die Zahl 2.

Als Halogenatome $R^3$ werden Fluor, Chlor und Brom verstanden. Bevorzugt wird das Chloratom.

Als Halogenatome Hal lassen sich alle Halogenatome verwenden, die unter den Bedingungen von Grignard-Reaktionen reagieren. Bevorzugt ist hier das Bromatom.

Als Halogenatome Hal' sind Chlor- Brom- und Jodatome zu verstehen.

Die Grignardverbindungen der allgemeinen Formel III werden in an sich bekannter Weise (zum Beispiel gemäß Houben-Weyl 13/2a, S. 63 ff oder Rosseels et al, Synthesis 1970, S. 302) aus den entsprechenden Alkylchloriden oder -bromiden mit Magnesium hergestellt.

Die Verbindungen der allgemeinen Formel IV lassen sich über entsprechende Triphenylphosphoniumbromide in ebenfalls an sich bekannter Weise herstellen (z.B. Jap. Patent 78, 150,000).

Die Überführung der freien Basen der allgemeinen Formel I in deren pharmakologisch verträgliche Salze erfolgt durch Neutralisation mit einer entsprechend anwendbaren organischen oder anorganischen Säure, wie z.B. Salzsäure, Schwefelsäure, Phosphorsäure, Bromwasserstoffsäure, Essigsäure, Fumarsäure, Oxalsäure, Milchsäure, Zitronensäure, Apfelsäure, Salicylsäure, Malonsäure, Maleinsäure, Bernsteinsäure, Weinsäure oder Ascorbinsäure. Zur Herstellung von Arzneimitteln werden die Wirkstoffe mit üblichen Zusätzen und flüssigen oder festen Trägerstoffen verarbeitet. Die Verbindungen der Formel III können in weiten Dosierungsgrenzen in flüssiger oder fester Form oral oder parenteral appliziert werden.

Übliche Zusätze für flüssige Formen sind z.B. Tartrat- und Citrat-Puffer, Äthanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls Geschmacks- und Süßstoffe enthalten.

Die erfindungsgemäß erhaltenen Verbindungen der allgemeinen Formel I besitzen außerordentlich günstige neuropharmakologische, insbesondere antidepressive Eigenschaften.

Die weite Verbreitung depressiver Erkrankungen ist statistisch gesichert. Studien des National Institute of Mental Health, Bethesda, USA, ergaben, daß etwa 16 % aller Erwachsener pathologisch-depressive Symptome aufweisen. In der Pharmakotherapie der Depression stellen die tricyclischen Antidepressiva die Mittel der ersten Wahl dar, wobei Amitryptilin und Imipramin die größte Verbreitung gefunden haben. Alle diese Antidepressiva besitzen schon im therapeutischen Bereich einschränkende anticholinerge Nebenwirkungen, wie Sedation, Mundtrockenheit, Konstipation, Benommenheit, Schwindel und Kopfschmerz sowie Tachykardie, gestörtes Sehvermögen, Harnverhaltung und persistierender Tremor. Die Auffindung neuartiger Antidepressiva mit verringerten kardiovaskulären und zentralen Nebenwirkungen bleibt deshalb ein wichtiges Ziel der medizinischen Chemie. Die pharmakologischen Ergebnisse mit den erfindungsgemäßen Verbindungen der Formel I weisen darauf hin, daß es sich hier um Antidepressiva mit einem bislang unbekannten psychopharmakologischen Profil handelt. Darüber hinaus eignen sich die erfindungsgemäßen Verbindungen überraschenderweise zur Therapie peptischer und duodenaler Ulcera, was insbesondere wegen der weitgehend fehlenden kardialen Nebenwirkungen, die für klassische Tricyclen leider typisch sind, von großer Bedeutung ist. Hierbei ist bemerkenswert, daß die unerwartete intestinale, antisekretorische Wirkung nicht auf anticholinergen Mechanismen beruht.

Die folgende Tabelle I belegt, daß die Verbindungen der allgemeinen Formel I sowie deren pharmakologisch verträgliche Salze mit anorganischen oder organischen Säuren antidepressive Eigenschaften ohne sedierende, anticholinerge und kreislaufbeeinträchtigende (kardiotoxische) Nebenwirkungen zu besitzen:

TABELLE I

| | Amitryptilin | Verbindungen gemäß Beispiel Nr. 1 | 4 a) | 4b) | 4c) |
|---|---|---|---|---|---|
| Toxizität LD50 mg/kg | 100 s.c.<br>200 i.g. | 300 i.g. | 200 i.g. | 200 i.g. | 800 i.g. |
| A | 5 mg/kg s.c. | 5 mg/kg s.c.<br>20 mg/kg i.g. | 5 mg/kg s.c.<br>20 mg/kg i.g. | 10 mg/kg s.c.<br>30 mg/kg i.g. | 75 mg/kg i.g. |
| B | 10 mg/kg s.c. | 10 mg/kg s.c. | 10 mg/kg s.c. | 20 mg/kg s.c. | 75 mg/kg i.g. |
| C | mg/kg s.c. | mg/kg s.c. | mg/kg s.c. | mg/kg s.c. | mg/kg i.g. |
| | 5 | 5 | 10 | 10 | 150 |
| K | 20 | 20 | 16 | 20 | 19 |
| M | 2 | 2 | 3 | 2 | 6 |
| K | 17 | 17 | 12 | 13 | 15 |
| S | 4 | 20 | 12 | 21 | 19 |
| D | 5 mg/kg s.c.<br>+ 103 % | 5 mg/kg s.c.<br>- 42 % | 5 mg/kg s.c.<br>± 0 % | 10 mg/kg s.c.<br>+ 11 % | 150 mg/kg i.g.<br>± 0 % |
| E | ED50 i.g.<br>23.0 mg/kg | bis 75 mg/kg s.c.<br>kein Effekt | bis 75 mg/kg s.c.<br>kein Effekt | bis 75 mg/kg s.c.<br>kein Effekt | bis 200 mg/kg i.g<br>kein Effekt |
| F | ED50 i.v.<br>1.0 mg/kg | kein Effekt | kein Effekt | kein Effekt | kein Effekt |

Legenden zur Tabelle

A) Die Umkehr der Reserpin-induzierten Hypothermie an Mäusen ist, nach Askew (Life Sci. 2, 725 (1963)) eine Untersuchungsmethode auf antidepressive Wirkung. Angegeben wird die Minimaldosis, welche zur Normalisierung der rektal gemessenen Körpertemperatur führt.

B) Antidepressiva antagonisieren die durch Tetrabenazin an der Maus erzeugte Antriebsarmut und Passivität gegenüber induzierenden Reizen sowie die Ausbildung und das Beibehalten abnormer Körperhaltungen (Arch. Int. Pharmacodyn. Ther. 115, 1 - 31 (1958)). Die Dosis, welche zur Normalisierung des Verhaltens führt, wird angegeben.

C) Die lokomotorische Aktivität gibt die stimulierenden oder depressiven Wirkungen auf das Zentralnervensystem wieder. Gemessen wurde die Spontanmotilität (Impulse pro Min.) an der Maus mittels photoelektrischer Systeme nach Irwin (Rev. Can. Biol. 20, 239 (1961)). In der Spalte bedeuten K Kontrolle (zweimalig), M Methaqualon (40 mg/kg i.g.) und S Substanz.

D) Die Schlafzeit-Verlängerung ist eine gängige in vivo Methode zur Bestimmung zentral dämpfender oder stimulierender Wirkungen. Versuchstiere sind Mäuse nach intravenöser (i.V.) Injektion von 82,5 mg/kg Hexobarbital. Der Einfluß auf die Schlafzeit unter der Prüfsubstanz wird in % zur Kontrolle angegeben.

## Nebenwirkungen

E) Anticholinerge Wirkung als Mydriasis gemessen (Maus).

F) Autonomes Testmodell am Hund nach i.v. Katecholamingabe. In Dosen, welche an sich noch keinen Blutdruckeffekt haben, potenzieren die tricyclischen Antidepressiva den Druckeffekt von Nor-adrenalin und Adrenalin.

Der folgende Vergleichsversuch belegt die Anti-Ulcus Wirkung der Verbindung des Beispiels 1:

25 mg/kg (i.g.) Substanz gemäß Beispiel 1 hemmen repräsentiv die Magensekretion in der Shay-Präparation (H. Shay et al, Gastroenterology 5, S. 43 - 61 (1945)) um 56 %.
80 mg/kg Cimetidin (i.g.) hemmen im selben Testsystem die Magensekretion um 45 %.

Die tierexperimentell gewonnenen Daten lassen erfahrungsgemäß zuverlässige therapeutische Effekte am Menschen je nach Indikation und Alter bei Dosen zwischen 20 und 200 mg pro Tag erwarten. Im Normalfall dürfte die Tagesdosis zwischen 40 und 150 mg liegen.

Die folgenden Beispiele dienen der näheren Erläuterung der Erfindung.

## Beispiel 1.

5-(2-Diethylaminoethoxy)-7H-dibenzo(a,c)cyclohepten-5-on(7)•hydrochlorid

Zu einer Suspension von 2,4 g 80prozentigem Natriumhydrid in 100 ml wasserfreiem Dimethylformamid (DMFA) wird bei 20 °C unter Stickstoffatmosphäre eine Lösung von 15 g (0,007 M) Dibenzo(a,c)cycloheptan-5,7-dion unter Turbinieren getropft. Nach 1,5 Stunden wird eine frisch bereitete Lösung von 14 g (∼ 0,1 M) Diethylaminoethylchlorid in 20ml DMFA tropfenweise zugefügt; anschließend läßt man bei 20 - 25 °C über 5 Stunden unter Rühren ausreagieren.

Das Produkt wird in Eiswasser eingetragen, mit Ether extrahiert und die etherische Phase zweimal mit Wasser gewaschen. Nach dem Entfernen des Lösungsmittels verbleibt ein Rückstand von 17,0 g, welcher in Ethylacetat aufgenommen und mittels Chlorwasserstoffgas als Hydrochlorid in feinkristalliner Form gefällt wird. Er wird aus Acetonitril umkristallisiert. Schmp. 210 - 212 °C.

## Beispiel 2

5-(N-Piperidinoethoxy)-7H-dibenzo(a,c)cyclohepten-5-on-(7)•hydrochlorid

In analoger Weise, wie in Beispiel 1 beschrieben, wird unter Verwendung von N-(2-Chlorethylpiperidin) verfahren. Das erhaltene Hydrochlorid von 5-(N-Piperidinoethoxy)-7H-dibenzo(a,c)cyclohepten-5-on-(7)• hydrochlorid wird aus Acetonitril umkristallisiert. Schmp. 222 - 223 °C.

0112584

## Beispiel 3

5-(N-Morpholinoethoxy)-7H-dibenzo(a,c)cyclohepten-5-on-(7)•hydrochlorid

Die Herstellung erfolgt in analoger Weise wie in Beispiel 1 beschrieben unter Verwendung von N-(2-Chlorethylmorpholin). Das Hydrochlorid von 5-(N-Morpholinoethoxy)-7H-dibenzo(a,c)cyclohepten-5-on-(7)•hydrochlorid wird aus Acetonitril/Ethanol = 3:2 umkristallisiert. Schm. 236 °C.

## Beispiel 4

5-(3-Dimethylaminopropyl)-7H-dibenzo(a,c)cyclohepten-5-on-(7) a)

### Variante A

Unter Stickstoffatmosphäre wird mittels 2,4 g (0,1 M) aktivierter Magnesiumspäne und 12,2 g (0,1 M) frisch destillierten Dimethylaminopropylchlorids in 60 ml wasserfreiem Banzol die entsprechende Grignard-Lösung hergestellt. Bei 50 °C tropft man eine Lösung von 8,4 g (0,04 M) Dibenzo(a,c)cycloheptan-5,7-dion unter Rühren innerhalb von 10 Minuten zu. Der Ansatz wird 3 Stunden unter Rückfluß gehalten und dann bei 20 °C mit einer Lösung von 24 g Ammoniumchlorid in 80 ml Wasser zersetzt. Die organische Phase wird abgetrennt, mit 2N Salzsäure extrahiert und der wäßrige Extrakt mittels Ammoniak auf pH 9 gestellt. Das abgeschiedene Produkt nimmt man in Ether auf; die Etherphase wird über Magnesiumsulfat getrocknet. Der Rückstand dieser Phase wird in Ethylacetat gelöst und mittels Oxalsäure gefällt. Das Oxalat von 5-(3-Dimethylaminopropyl)-7H-dibenzo(a,c)cyclohepten-5-on-(7) kristallisiert man aus Ethylmethylketon um. Schmp. 162 - 163 °C.

### Variante B

Aus 50 g Triphenylphosphin und 38,5 g 1,3-Dibrompropan in 150 ml Xylol stellt man durch 20stündiges Erhitzen auf 130 °C unter Stickstoff 80 g kristallines (3-Brompropyl)-triphenylphosphoniumbromid vom Schmp. 226 °C her. Dieses wird mittels einer Lösung von 33 g (0,7 M) Dimethylamin in 200 ml Ethanol 20 Stunden bei 20 - 25 °C gerührt. Der Rückstand nach dem Entfernen des Ethanols im Vakuum wird in 2-Propanol aufgenommen und mittels Bromwasserstoffgas gefällt. Nach dem Umkristallisieren aus Ethanol erhält man 43 g (3-Dimethylaminopropyl)-triphenylphosphoniumbromid HBr vom Schmp. 279 °C.

27 g (0,052 M) dieser Zwischenverbindung werden in 80 ml trockenem Tetrahydrofuran (THF) suspendiert und mittels 65 ml 15 %iger Butyllithiumlösung ( 0,105 M) bei 30 - 35 °C in das Ylid überführt. Nach der Zugabe von 10,5 g (0,05 M)Dibenzo(a,c)cycloheptan-5,7-dion in 80 ml THF bei 30 °C wird 3 Stunden unter Rückfluß erhitzt. Der Ansatz wird mittels 15 ml Wasser zersetzt und das Lösungsmittel am Rotavapor entfernt. Der Rückstand liefert mit 2N Salzsäure behandelt ein in Salzsäure schwerlösliches, kristallines Hydrochlorid, das aus Ethanol umkristallisiert wird. Schmp. 209 °C.

In analoger Weise erhält man folgende Verbindungen:

5-(3-Diethylaminopropyl)-7H-dibenzo(a,c)cyclohepten-5-on-(7)• oxalat, b) Schmp. 125 - 126 °C (Ethylmethylketon / 2-Propanol 1:1)

5-(3-Methylaminopropyl)-7H-dibenzo(a,c)cyclohepten-5-on-(7)• oxalat, c) Schmp. 169 - 170 °C (Ethanol)

5-(3-(4-Methyl-1-piperazino)-propyl)-7H-dibenzo(a,c)cyclohepten-5-on-(7)• dioxalat, d) Schmp. 217 - 218 °C (Ethanol/Wasser 5:1)

Variante C

5-(3-Dimethylaminopropyl)-7H-dibenzo(a,c)cyclohepten-5-on-(7)• hydrochlorid

Die Umsetzung des Ylides nach Variante B kann auch mit einem Enolether des Dibenzo(a,c)cycloheptan-5,7-diones erfolgen. Den Ethylenolether stellt man wie folgt her:

Eine Lösung von 56 g (0,25 M) Dibenzo(a,c)cycloheptan-5,7-dion in 900 ml Ethanol wird mit 42 g (0,3 M) Kaliumcarbonat versetzt. Unter Turbinieren tropft man eine Lösung von 37,5 ml Diethylsulfat in 80 ml Ethanol innerhalb von 30 Minuten bei 50 °C ein. Es wird 4 Stunden zum Rückfluß erhitzt. Nach dem Abkühlen gibt man auf Eis und trennt den kristallinen Niederschlag ab, welcher nach dem Trocknen aus Ethanol umkristallisiert wird. Schmp. 133 °C. Die Umsetzung des 5-Ethoxy-7-H-dibenzo(a,c)cyclohepten-5-on-(7) mit dem Ylid erfolgt wie in Variante B beschrieben.

## Beispiel 5

3-Chlor-5-(3-diethylaminopropyl)-7H-dibenzo(a,c)cyclohepten-5-on-(7) oxalat

145 g (0,6 M) Diphensäure und 93,5 g (0,3 M) $Ag_2SO_4$ werden in eine Mischung aus 1,2 l konzentrierter Schwefelsäure und 120 ml Wasser unter Rühren gelöst.

Danach leitet man 42,0 g gasförmiges Chlor ein (Dauer ca. 15 Stunden). Es wird auf reichlich Eis gegeben, das organische Produkt abgetrennt und mit 1 l siedendem Ethanol extrahiert. Nach der Konzentration der Ethanolphase auf 400 ml wird mit 800 ml heißem Wasser verdünnt. Man erhält 122 g 4-Chlor-diphenyl-1,1'-dicarbonsäure. Schmp. 260 °C. (Benzol).

71 g (0,27 M) 4-Chlor-diphenyl-1,1'-dicarbonsäure werden mit 900 ml Triethylamin auf 90 °C erhitzt und innerhalb von 3 Stunden mit 57 g (0,35 M) Malonsäurediethylester versetzt. Nach 2,5 Stunden bei 100 °C wird abgekühlt und das Produkt in Eiswasser eingetragen. Die Oberschicht wird abdekantiert und der Rückstand aus 2-Propanol umkristallisiert. 66 g (Schmp. 80 °C) an o-Diethylmalonyliden-Derivat des 4-Chlor-diphenyl-1,1'-dicarbonsäureanhydrides.

41 g (0,11 M) 4-Chlor-diphenyl-1,1'-dicarbonsäureanhydrid werden in 230 ml Ameisensäure unter Zusatz von 10 ml Wasser 24 Stunden zum Rückfluß erhitzt. Das nach dem Eintragen in Eiswasser abgeschiedene Öl kristallisiert. 30 g 1'-Acetyl-4-chlor-diphenyl-1-carbonsäure vom Schmp. 132 °C (Benzol/Cyclohexan 1:1).

2,6 g Natrium (0,11 M) werden in 150 ml wasserfreiem Ethanol gelöst. Man trägt 26 g (0,1 M) der obigen Verbindung ein und cyclisiert durch 6stündiges Erhitzen unter Rückfluß. Durch Eintragen in 0,7 l Eiswasser und Versetzen mit 2 N Salzsäure bis pH 4 erhält man 23,6 g

3-Chlor-dibenzo(a,c)cycloheptan-5,7-dion vom Schmp. 210 - 215 °C. Sie ist zur Weiterverarbeitung hinreichend rein.

23 g (0,09 M) 3-Chlor-dibenzo(a,c)cycloheptan-5,7-dion werden in 400 ml Ethanol gelöst und mittels 15,5 g (0,11 M) Kaliumcarbonat und 15 ml Diethylsulfat analog Beispiel 4, Variante C, in den entsprechenden Enolether überführt. Schmp. 114 °C (Cyclohexan).

Unter Stickstoff werden 7,7 g (0,015 M) (3-Dimethylaminopropyl)-triphenylphosphoniumbromid HBr analog Beispiel 4, Variante B, hergestellt und in das Ylid überführt. Nach der Zugabe von 3,1 g (0,011 M) 3-Chlor-5-ethoxy-7H-dibenzo(a,c)cyclohepten-5-on-(7), gelöst in 25 ml THF wird noch 3 Stunden unter Rückfluß erhitzt. Zersetzung und Aufarbeitung wie unter Beispiel 4, Variante B, beschrieben. Das Oxalat wird aus Ethylacetat gefällt und aus 2-Propanol umkristallisiert. Man erhält 3-Chlor-5-(3-diethylaminopropyl)-7H-dibenzo(a,c)cyclohepten-5-on-(7)•oxalat vom Schmp. 162 - 165 °C.

Patentansprüche

1.) 7H-Dibenzo(a,c)cyclohepten-5-on(7)-Derivate der allgemeinen Formel I

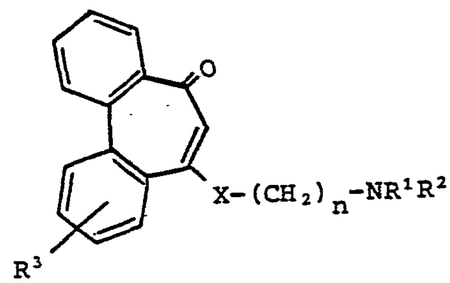

(I),

worin $R^1$ und $R^2$ gleich oder verschieden sein können und ein Wasserstoffatom, eine Alkylgruppe mit 1 - 3 Kohlenstoffatomen oder zusammen mit dem Stickstoffatom an das sie gebunden sind, einen heterocyclischen Ring mit 3 - 6 Kohlenstoffatomen,

n die Zahl 2 oder 3

$R^3$ ein Wasserstoffatom oder ein Halogenatom und

X entweder eine Methylengruppe oder ein Sauerstoffatom bedeutet

sowie deren pharmakologisch verträgliche Salze mit anorganischen oder organischen Säuren.

2.) Verbindungen gemäß Anspruch 1, in welchen der heterocyclische Ring mit 3 - 6 Kohlenstoffatomen einen Piperidin-, Morpholin- oder N-Alkylpiperazinring bedeutet.

3.) Verbindungen gemäß Anspruch 1, worin $R^1$ und/oder $R^2$ ein Wasserstoffatom einen Methyl- oder Ethylrest bedeutet.

4.) Verfahren zur Herstellung von Verbindungen der allgemeinen Formel I sowie von deren pharmakologisch verträglichen Salzen, dadurch gekennzeichnet, daß man eine Verbindung der allgemeinen Formel II

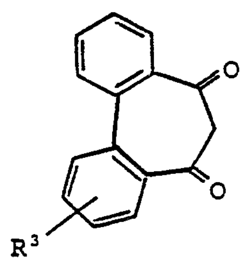

(II),

in welcher $R^3$ die oben angegebene Bedeutung hat

in an sich bekannter Weise entweder

a) mit Grignard-Verbindungen der allgemeinen Formel III

$$Hal\ Mg\text{-}(CH_2)_n\text{-}NR^1R^2 \quad (III),$$

in welcher Hal ein Halogenatom bedeutet und n, $R^1$ und $R^2$ die obengenannte Bedeutung haben oder

b) mit Verbindungen der allgemeinen Formel IV

$$(C_6H_5)_3P\text{=}CH\text{-}(CH_2)_{n-1}\text{-}NR^1R^2 \quad (IV)$$

in welcher n, $R^1$ und $R^2$ die obengenannte Bedeutung haben oder

c) für den Fall, daß X eine Methylengruppe sein soll, indem man Verbindungen der allgemeinen Formel V

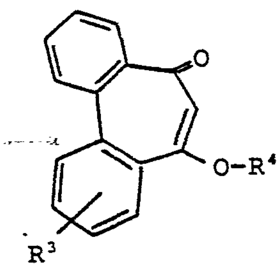

(V),

in welcher $R^3$ die obengenannte Bedeutung hat und $R^4$ einen Alkylrest mit 1 - 3 Kohlenstoffatomen bedeutet mit Verbindungen der allgemeinen Formel IV umsetzt oder

d) für den Fall, daß X ein Sauerstoffatom sein soll, indem man Verbindungen der allgemeinen Formel II zunächst mittels starker Basen in die entsprechenden Enolate überführt und diese mit Verbindungen der allgemeinen Formel VI

$$Hal'-(CH_2)_n-NR^1R^2 \qquad (VI)$$

in welcher Hal' ein Halogenatom bedeutet und $R^1$ und $R^2$ die oben angegebene Bedeutung hat,

umsetzt und gewünschtenfalls anschließend mittels anorganischen oder organischen Säuren in deren pharmakologisch verträgliche Salze überführt.

5.) Arzneimittel enthaltend mindestens ein 7H-Dibenzo(a,c)cyclohepten-5-on(7)-Derivat der allgemeinen Formel I zur Bekämpfung psychischer Erkrankungen und von Magen- und/oder Darmgeschwüren.